# EUROPEAN PATENT APPLICATION

(11) **EP 1 612 269 A1**
(43) Date of publication of application: **04.01.2006**
(21) Application number: 05009308.7
(22) Date of filing: 21.09.1999
(51) Int. Cl.: C12N 15/30, A61K 39/00, A61K 39/015

(54) **Use of replication-deficient adenoviral vector to boost CD8+ T cell immune response to antigen**

(30) Priority: 21.09.1999 GB 9922361
(62) Divisional of application: 00960857.1
(71) Applicant: ISIS INNOVATION LIMITED, Summertown, Oxford OX2 7SG (GB)
(72) Inventor: Schneider, Joerg, Oxford OX3 8BT (GB); Gilbert, Sarah Catherine, Headington Oxford OX3 7B (GB); Hannan, Carolyn Mary, Rosanna Victoria 3084 (AU); Hill, Adrian Vivian Sinton, Oxford OX3 9DL (GB)
(74) Representative: Walton, Seán Malcolm

(57) **Abstract**

Use of a replication-deficient adenoviral vector encoding an antigen or a CD8+ T cell epitope of the antigen to boost in the individual a CD8+ T cell immune response to the antigen following prior administration of a priming composition. The priming composition comprises the antigen or epitope or nucleic acid encoding the antigen or epitope, and may be DNA, Ty-LVP'S or Modified Virus Ankara (MVA). Administration may b e intradermal or intramucular.

## Description

The present invention relates to generation of a CD8+ T cell immune response against an antigen. More particularly, the present.invention relates to "prime and boost" immunisation regimes in which the immune response induced by administration of a priming composition is boosted by administration of a boosting composition. The present invention is based on the inventors' experimental demonstration that effective boosting can be achieved using replication-defective adenovirus vectors, following priming with any of a variety of different types of priming compositions.

A major protective component of the immune response against a number of pathogens is mediated by T lymphocytes of the CD8+ type, also known as cytotoxic T lymphocytes (CTL). An important function of CD8+ cells is secretion of gamma interferon (IFNγ), and this provides a measure of CD8+ T cell immune response.

CD8+ T cell response is important in protection against a number of parasites, including protozoan parasites such as *Toxoplasma* and *Trypanosoma, Plasinodium falciparum* (and in mice *P. berghei*)*,* viruses such as HIV, herpes simplex, herpes zoster, HBV, HCV influenza, EBV, measles, dengue and HTLV-1, bacteria such as *Mycobacterium tuberculosis* and *Listeria* sp, and various cancers, such as melanoma and renal carcinoma.

Infection of mice with *P*. *berghei* provides a good model for *P*. *falciparum* malaria in humans, chosen by the inventors for exemplification of the ability of the present invention to provide strong CD8+ T cell immune responses against antigen. Malaria is a major health problem in the world and significant efforts have been directed in finding effective immune compositions for vaccination.

In order to protect against the pre-erythrocytic stage of *Plasmodium falciparum* malaria, an immunogenic composition must induce a strong CD8+ T cell response. In general, live attenuated vaccines capable of producing a short-lived infection that is harmless in a healthy individual are effective at inducing T cell responses. Irradiated *Plasmodium* sporozoites, which can infect hepatocytes but do not progress to a blood-stage infection have been shown to protect both mice and men against malaria by inducing.T cell responses against pre-erythrocytic antigens [Nardin and Nussenzweig (1993) Annu. Rev. Immunol. 11: 687-727]. However in man this requires multiple immunisations over a long period of time, and whilst these experimental immunisations have provided valuable information, this approach is not useful for development of a vaccine.

Recombinant protein subunit vaccines elicit humoral responses, but poor CD8+ T cell responses [Schirmbeck et al., (1995) Vaccine 13(9): 857-865]. DNA vaccines have been shown to elicit both humoral and cellular responses against the *P*. *yoelii* CS protein [Sedegah et al., (1994) Proc. Natl. Acad. Sci. USA 91 (21) : 9866-70]. However, mice immunised with a DNA vaccine expressing the *P*. *berghei* CS gene had only a weak CD8+ T cell response to the protective CD8+ T cell epitope pb9 [Romero et al., (1989) Nature 341(6240): 323-6] and were not protected against challenge with infectious sporozoites even after repeated immunisations [Schneider et al., (1998) Nat. Med. 4(4): 397-402]. Ty virus-like particles, consisting of a recombinant protein assembled into a 30nm particle, induce stronger CD8+ T cell responses, but do not protect against infection [Gilbert et al., (1997) Nat. Biotechnol. 15(12): 1280-4].

Recombinant viruses can also be used as vaccines. Modified Vaccinia virus Ankara (MVA) does not replicate in human cells and is a very safe virus to use as a vaccine [Mayr et al., (1978) Zentralbl. Bakteriol. 167(5-6): 375-90; Sutter and Moss (1992) Proc. Natl. Acad. Sci. 89 (22): 10847-51; Sutter et al., (1994) Vaccine 12(11): 1032-40]. Recombinant MVA expressing *P*. *berghei* CS has also been tested in mice, resulting in similar levels of peptide-specific lysis in a cytotoxicity assay as mice immunised with Ty VLPs [Gilbert et al., (1999) Biol. Chem. 380 (3): 299-303]. Again, these mice were not protected against infectious challenge. However, despite the fact that neither DNA vaccines, Ty VLPs or MVA used alone can protect against malaria infection, using either DNA or Ty VLPs to prime a T cell response and MVA to boost it results in greatly increased numbers of IFN-γ secreting CD8+ T cells, and complete protection against infection when the MVA is administered intravenously [Schneider et al., (1998) Nat. Med. 4 (4): 397-402, WO98/56919]:

The present invention employs replication-deficient adenovirus which, as the experiments described below show, has been found to be an effective means for providing a boost to a CD8+ T cell immune response primed to antigen using any of a variety of different priming compositions.

Replication-deficient adenovirus derived from human serotype 5 has been developed as a live viral vector by Graham and colleagues [Graham and Prevec (1995) Mol. Biotechnol. 3(3): 207-20; Bett et al., (1994) Proc. Natl. Acad. Sci. USA 91(19): 8802-6]. Adenoviruses are non-enveloped viruses containing a linear double stranded DNA genome of around 3600 bp. Recombinant viruses can be constructed by *in vitro* recombination between an adenovirus genome plasmid and a shuttle vector containing the gene of interest together with a strong eukaryotic promoter, in a permissive cell line which allows viral replication. High viral titres can be obtained from the permissive cell line, but the resulting viruses, although capable of infecting a wide range of cell types, do not replicate in any cells other than the permissive line, and are therefore a safe antigen delivery system. Recombinant adenoviruses have been shown to elicit protective immune responses against a number of antigens including tick-borne encephalitis virus NS1 protein [Jacobs et al., (1992) J. Virol. 66(4): 2086-95] and measles virus nucleoprotein [Fooks et al., (1995) Virology 210(2): 456-65]. Further, a single dose of recombinant adenovirus resulted in a 93% decrease in the level of hepatic parasite rRNA of *P. yoelii* in mice, and 40% protection, which was shown to be CD8+ T cell-mediated [Rodrigues et al., (1997) J. Immunol. 158 (3) : 1268-74] .

Remarkably, the experimental work described below demonstrates that use of embodiments of the present invention allows for recombinant replication-defective adenovirus expressing an antigen (specifically exemplified with the CS gene from *Plasmodium berghei*) to boost a CD8+ T cell immune response primed by a DNA vaccine, Ty-VLPs or recombinant modified Vaccinia virus Ankara (MVA). The replication-defective adenovirus was found to induce a CD8+ T cell response after intradermal or intramuscular immunisation. In prime/boost vaccination regimes the replication-defective adenovirus was also able to prime a response that could be boosted by MVA.

Mice immunised with the replication-defective adenovirus and boosted with the MVA were completely protected against *P*. *berghei* sporozoite challenge. Both recombinant replication-deficient adenovirus and recombinant MVA are vaccines that are safe for use in humans. Advantageously, the inventors found that a vaccination regime used intradermal immunisation for both prime and boost can be employed, constituting a general immunisation regime suitable for inducing CD8+ T cells, e.g. in humans.

The present invention in various aspects and embodiments employs a replication-deficient adenovirus vector encoding an antigen for boosting a CD8+ T cell immune response to the antigen primed by previous administration of the antigen or nucleic acid encoding the antigen.

A general aspect of the present invention provides for the use of a replication-deficient adenoviral vector for boosting a CD8+ T cell immune response to an antigen.

One aspect of the present invention provides a method of boosting a CD8+ T cell immune response to an antigen in an individual, the method including provision in the individual of a replication-deficient adenoviral vector including nucleic acid encoding the antigen operably linked to regulatory sequences for production of antigen in the individual by expression from the nucleic acid, whereby a CD8+ T cell immune response to the antigen previously primed in the individual is boosted.

An immune response to an antigen may be primed by immunisation, by infection with an infectious agent, or by development of a tumour or malignancy.

A further aspect of the invention provides a method of inducing a CD8+ T cell immune response to an antigen in an individual, the method comprising administering to the individual a priming composition comprising the antigen or nucleic acid encoding the antigen and then administering a boosting composition which comprises a replication-deficient adenoviral vector including nucleic acid encoding the antigen operably linked to regulatory sequences for production of antigen in the individual by expression from the nucleic acid.

A further aspect provides for use of a replication-deficient adenoviral vector, as disclosed, in the manufacture of a medicament for administration to a mammal to boost a CD8+ T cell immune response to an antigen. Such a medicament is generally for administration following prior administration of a priming composition comprising the antigen.

The priming composition may comprise any viral vector, although generally other than adenoviral, such as a vaccinia virus vector such as a replication-deficient strain such as modified virus ankara (MVA) (Mayr et al., (1978) Zentralbl. Bakteriol. 167(5-6): 375-90; Sutter and Moss (1992) Proc. Natl. Acad. Sci. 89(22): 10847-51; Sutter et al., (1994) Vaccine 12(11): 1032-40) or NYVAC (Tartaglia et al., Virology (1992) 118 (1) : 217-32), an avipox vector such as fowlpox or canarypox, e.g. the strain known as ALVAC (Kanapox, Paoletti et al., *Dev Biol Stand* (1994) 82: 65-9), or a herpes virus vector. The priming composition may comprise a recombinant bacterial vector, such as recombinant BCG or *Salmonella.* A priming composition comprising a recombinant fowlpox virus is among preferred embodiments for use in the present invention.

The priming composition may comprise DNA encoding the antigen, such DNA preferably being in the form of a circular plasmid that is not capable of replicating in mammalian cells Any selectable marker should not be resistance to an antibiotic used clinically, so for example Kanamycin resistance.is preferred to Ampicillin resistance. Antigen expression should be driven by a promoter which is active in mammalian cells, for instance the cytomegalovirus immediate early (CMV IE) promoter.

The priming composition may be a recombinant Ty-VLP. These are protein particles consisting of a single protein species from the Tyl retrotransposon of *S*. *cerevisiae* which spontaneously assembles into particles. Recombinant Ty-VLP's may be produced by fusing the coding sequence of the required epitope or antigen to the 3' end of the coding sequence for the TyA protein, and transforming *S*. *cerevisiae* with a vector including the coding sequence for expression of the fusion protein which then assembles into particles in the yeast cytoplasm from where they may be purified. The particulate nature of the Ty-VLP's allows them to be taken up by antigen presenting cells and prime a CD8+ T cell response to epitopes contained within them.

Other suitable priming compositions include lipid-tailed peptides, fusion proteins, adjuvant compositions and so on.

In particular embodiments of the various aspects of the present invention, administration of a priming composition is followed by boosting with first and second boosting compositions, the first and second boosting compositions being different from one another, e.g. as exemplified below. Still further boosting compositions may be employed without departing from the present invention. In one embodiment, a triple immunisation regime employs DNA, then adenovirus as a first boosting composition, and then MVA as a second boosting composition, optionally followed by a further (third) boosting composition or subsequent boosting administration of one or other or both of the same or different vectors. Another option is DNA then MVA then Ad, optionally followed by subsequent boosting administration of one or other or both of the same or different vectors.

The antigen to be included in respective priming and boosting compositions (however many boosting compositions are employed) need not be identical, but should share at least one CD8+ T cell epitope. The antigen may correspond to a complete antigen in a target pathogen or cell, or a fragment thereof. Peptide epitopes or artificial strings of epitopes may be employed, more efficiently cutting out unnecessary protein sequence in the antigen and encoding sequence in the vector or vectors. One or more additional epitopes may be included, for instance epitopes which are recognised by T helper cells, especially epitopes recognised in indivuals of different HLA types (such as tetanus epitopes).

Within the replication-deficient adenoviral vector, regulatory sequences for expression of the encoded antigen will include a promoter. By "promoter" is meant a sequence of nucleotides from which transcription may be initiated of DNA operably linked downstream (i.e. in the 3' direction on the sense strand of double-stranded DNA). "Operably linked" means joined as part of the same nucleic acid molecule, suitably positioned and oriented for transcription to be initiated from the promoter. DNA operably linked to a promoter is "under transcriptional initiation regulation" of the promoter. Other regulatory sequences including terminator fragments, polyadenylation sequences, enhancer sequences, marker genes and other sequences may be included as appropriate, in accordance with the knowledge and practice of the ordinary person skilled in the art: see, for example, Molecular Cloning: a Laboratory Manual: 2nd edition, Sambrook et al., 1989, Cold Spring Harbor Laboratory Press. Many known techniques and protocols for manipulation of nucleic acid, for example in preparation of nucleic acid constructs, mutagenesis, sequencing, introduction of DNA into cells and gene expression, and analysis of proteins, are described in detail in Current Protocols in Molecular Biology, Ausubel et al. eds., John Wiley & Sons, 1994.

Suitable promoters for use in aspects and embodiments of the present invention include the cytomegalovirus immediate early (CMV IE) promoter, with or without intron A, and any other promoter that is active in mammalian cells.

Either or both of the priming and boosting compositions may include an adjuvant, such as granulocyte macrophage-colony stimulating factor (GM-CSF) or encoding nucleic acid therefor.

Administration of the boosting composition is generally about 10 days to 4 weeks after administration of the priming composition, preferably about 2-3 weeks.

Preferably, administration of priming composition, boosting composition, or both priming and boosting compositions, is intradermal or intramuscular immunisation

Intradermal administration of adenovirus and MVA vaccines may be achieved by using a needle to inject a suspension of the virus. An alternative is the use of a needleless injection device to administer a virus suspension (using e.g. Biojector™) or a freeze-dried powder containing the vaccine (e.g. in accordance with techniques and products of Powderject), providing for manufacturing individually prepared doses that do not need cold storage. This would be a great advantage for a vaccine that is needed in rural areas of Africa.

Adenovirus and MVA are both viruses with an excellent safety record in human immunisations. The generation of recombinant viruses can be accomplished simply, and they can be manufactured reproducibly in large quantities. Intradermal administration of recombinant replication-deficient adenovirus followed by recombinant MVA is therefore highly suitable for prophylactic or therapeutic vaccination of humans against diseases which can be controlled by a CD8+ T cell response.

The individual may have a disease or disorder such that delivery of the antigen and generation of a CD8+ T cell immune response to the antigen is of benefit or has a therapeutically beneficial effect.

Most likely, administration will have prophylactic aim to generate an immune response against a pathogen or disease before infection or development of symptoms.

Diseases and disorders that may be treated or prevented in accordance with the present invention include any noted already above and others in which a CD8+ T cell immune response may play a protective or therapeutic role.

Components to be administered in accordance with the present invention may be formulated in pharmaceutical compositions. These compositions may comprise a pharmaceutically acceptable excipient, carrier, buffer, stabiliser or other materials well known to those skilled in the art. Such materials should be non-toxic and should not interfere with the efficacy of the active ingredient. The precise nature of the carrier or other material may depend on the route of administration, e.g. intravenous, cutaneous or subcutaneous, nasal, intramuscular, intraperitoneal routes.

As noted, administration is preferably intradermal, subcutaneous or intramuscular.

Liquid pharmaceutical compositions generally include a liquid carrier such as water, petroleum, animal or vegetable oils, mineral oil or synthetic oil. Physiological saline solution, dextrose or other saccharide solution or glycols such as ethylene glycol, propylene glycol or polyethylene glycol may be included.

For intravenous, cutaneous or subcutaneous injection, or injection at the site of affliction, the active ingredient will be in the form of a parenterally acceptable aqueous solution which is pyrogen-free and has suitable pH, isotonicity and stability. Those of relevant skill in the art are well able to prepare suitable solutions using, for example, isotonic vehicles such as Sodium Chloride Injection, Ringer's Injection, Lactated Ringer's Injection. Preservatives, stabilisers, buffers, antioxidants and/or other additives may be included, as required.

A slow-release formulation may be employed.

Following production of replication-deficient adenoviral particles and optional formulation of such particles into compositions, the particles may be administered to an individual, particularly human or other primate.
Administration may be to another mammal, e.g. rodent such as mouse, rat or hamster, guinea pig, rabbit, sheep, goat, pig, horse, cow, donkey, dog or cat.

Administration is preferably in a "prophylactically effective amount" or a "therapeutically effective amount" (as the case may be, although prophylaxis may be considered therapy), this being sufficient to show benefit to the individual. The actual amount administered, and rate and time-course of administration, will depend on the nature and severity of what is being treated. Prescription of treatment, e.g. decisions on dosage etc, is within the resporisibility of general practitioners and other medical doctors, or in a veterinary context a veterinarian, and typically takes account of the disorder to be treated, the condition of the individual patient, the site of delivery, the method of administration and other factors known to practitioners. Examples of the techniques and protocols mentioned above can be found in Remington's Pharmaceutical Sciences; 16th edition, Osol, A. (ed), 1980.

In one preferred regimen, DNA is administered (preferably intramuscularly) at a dose of 0.5 mg/injection, follwed by adenovirus (preferably intramuscularly or intradermally) at a dose of 5 x 10⁷ - 5 x 10⁸ virus particles/injection.

A composition may be administered alone or in combination with other treatments, either simultaneously or sequentially dependent upon the condition to be treated.

Delivery to a non-human mammal need not be for a therapeutic purpose, but may be for use in an experimental context, for instance in investigation of mechanisms of immune responses to an antigen of interest, e.g. protection against cancers, malaria, other pathogens and so on.

Further aspects and embodiments of the present invention will be apparent to those of ordinary skill in the art, in view of the above disclosure and following experimental exemplification, included by way of illustration and not limitation, and with reference to the attached figures, wherein:

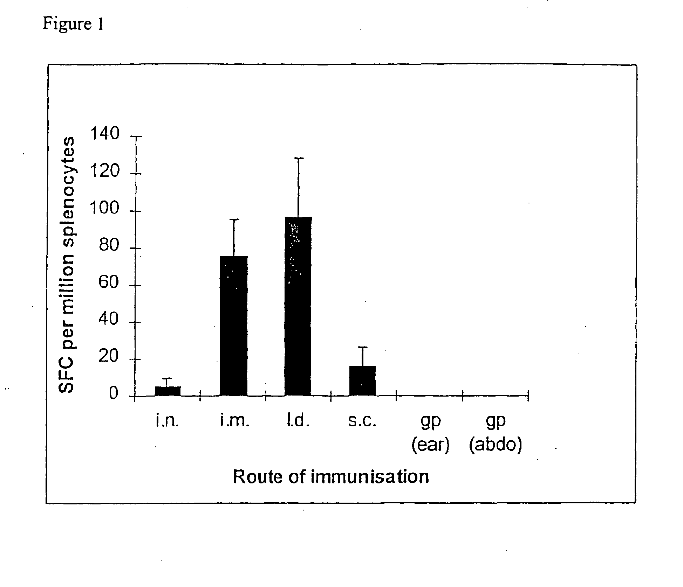

Figure 1 shows results of experiments demonstrating peptide-specific IFN-γ secreting T cells primed by a single immunisation of Ad-PbCS. Groups of three mice were immunised via the routes shown using 10⁷ pfu. Elispot assays to detect IFN-γ secreting pb9-specific T cells were performed in duplicate on splenocytes after two weeks. The graph shows spot forming cells (SFC) per million splenocytes for each route of administration.

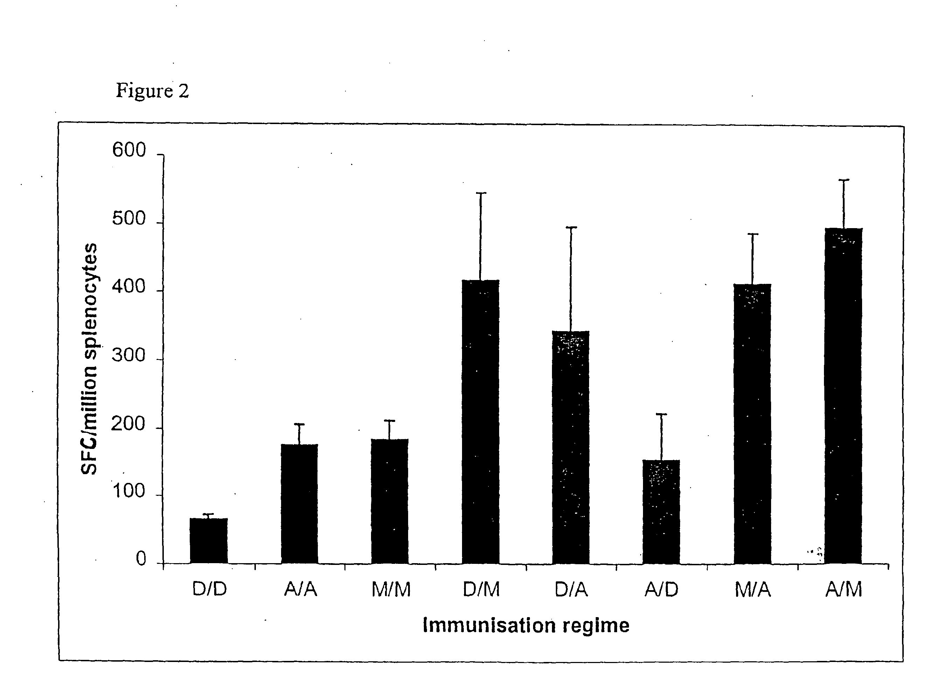

Figure 2 shows results of prime/boost immunisations. Groups of three mice were immunised on day 0 with the first vaccine shown (D = pSG2.PbCS, A = Ad-PbCS, M = MVA-PbCS), and day 14 with the second vaccine. DNA was injected i.m., adenovirus and MVA i.d. Elispots were performed on splenocytes isolated on day 28. The graph shows SFC per million splenocytes for each prime/bosst immunisation regime.

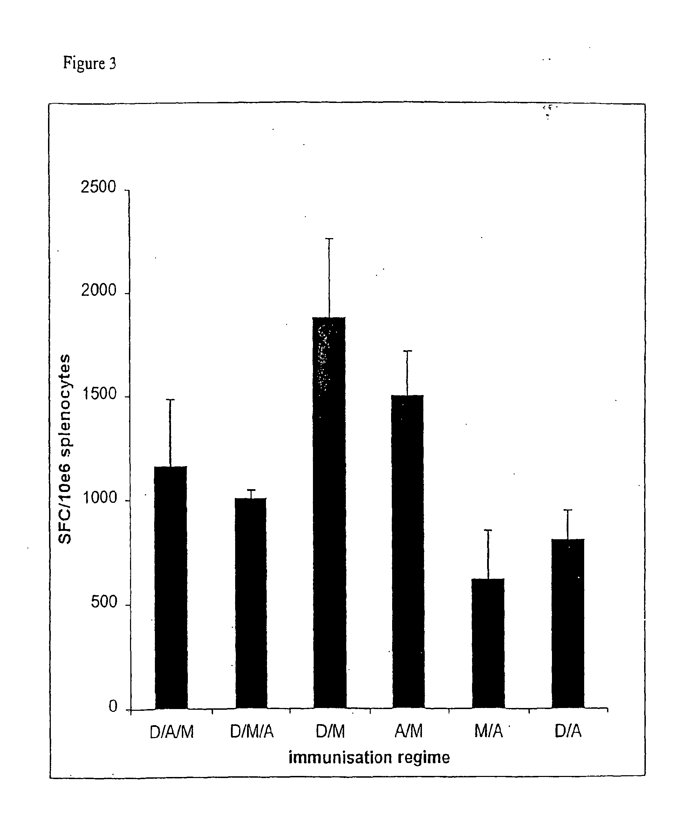

Figure 3 shows results of triple combination immunisations, and comparison with double combination immunisations. Groups of three mice were immunised at 10 day intervals with the vaccines shown (D = pSG2.PbCS, A= Ad-PbCS, M = MVA-PbCS), with the first vaccine of the double combinations given on the same day as the second vaccine of the triple combinations. Elispot assays were performed in duplicate on splenocytes 10 days after the last immunisation. The graph shows SFC per million splenocytes for each immunisation regime.

### EXPERIMENTAL EXEMPLIFICATION

The inventors constructed a recombinant replication deficient adenovirus expressing the CS gene of *P*. *berghei,* (Ad-PbCS) and tested the capabilities of this virus to induce CD8+ T cell responses in mice either alone or in combination with other types of vaccines.

When used as a single immunisation high levels of antigen-specific CD8+ T cells were generated. Adenovirus priming followed by MVA boosting resulted in complete protection.

Remarkably, adenovirus was able to boost substantially a response primed by DNA, Ty-VLPs or MVA.

### MATERIALS AND METHODS

### DNA vaccine

The DNA vaccine pSG2.PbCS consists of the CMV promoter with intron A driving expression of *P. berghei* CS protein, with the bovine growth hormone poly A sequence. The plasmid is kanamycin resistant and incapable of replication in eukaryotic cells. Plasmids were prepared using Qiagen columns and diluted in endotoxin-free phosphate buffered saline (PBS).

### Construction of recombinant replication deficient adenovirus

The CMV promoter with intron A, *P. berghei* CS protein gene and bovine growth hormone poly A sequence from pSG2.PbCSP was ligated into the multiple cloning site of the adenovirus shuttle vector pΔE1sp1A [Bett et al., (1994) Proc. Natl. Acad. Sci. USA 91(19): 8802-6]. This vector can be used to construct Adenovirus 5 recombinants with deletions in E1. The recombinant shuttle vector was used to transfect the permissive cell line 293 along with the adenovirus genome plasmid pJM17 [Graham and Prevec (1995) Mol. Biotechnol. 3(3): 207-20]. Virus from transfected cells was clonally purified by three successive limiting dilutions in 293 cells, and expression of *P. berghei* CS in cells infected with the isolated virus was confirmed by immunofluoresence. Large quantities of virus were prepared from infected 293 cells and purified by extraction with Arklone [Graham and Prevec (1995) Mol. Biotechnol. 3(3): 207-20] prior to immunisation.

### Ty VLPs

Recombinant Ty VLPs expressing the pb9 epitope from *P*. *berghei* CS, SYIPSAEKI, were prepared as described in [Gilbert et al., (1997) Nat. Biotechnol. 15(12): 1280-4] and suspended in PBS.

### Recombinant MVA

MVA expressing *P. berghei* CS was prepared by *in vitro* recombination between a shuttle vector containing the CS gene driven by the vaccinia P7.5 promoter and MVA virus in primary chick embryo fibroblasts [Sutter et al., (1994) Vaccine 12(11): 1032-40]. The recombinant, which also expresses *E*. *coli* β-galactosidase, was repeatedly plaque purified and expression of the recombinant gene was confirmed by immunofluoresence. Virus for immunisation was purified by ultracentrifugation through a sucrose cushion and suspended in endotoxin-free PBS.

### Immunisations

Female BALB/c mice 4-6 weeks old were immunised under anaesthesia as described for individual experiments. Intramuscular DNA immunisations used 50 µg DNA in each musculus tibialis. MVA and adenovirus (10⁶ and 10⁷ pfu per dose respectively) were injected intradermally into the ear pinae. Ty VLPs (100 µg per dose) were injected intradermally in the footpad or intravenously into the lateral tail vein.

### ELISPOT assays

The number of IFN-γ secreting, pb9-specific T cells in fresh splenocyte preparations was determined as described previously [Schneider et al., (1998) Nat. Med. 4(9): 397-402] by coating 96-well nitrocellulose plates with anti-mouse IFN-γ antibody (clone R4 from ETCC), washing with PBS and subsequent blocking with complete medium containing 10% FCS. Splenocytes from' immunised mice were resuspended at 1-2 x 10⁷ cells/ml and placed in duplicates into the coated wells, and serially diluted. The H2-K^{d} -restricted peptide pb9 (SYIPSAEKI) (Romero) was added to test wells and an irrelevant peptide to control wells. After overnight incubation the wells were washed and a second, biotinylated anti-IFN-γ antibody (Pharmingen clone) added to the wells. The wells were washed again and streptavidin-alkaline phosphatase was added. After further washing, spots were developed by adding an alkaline phosphatase substrate. The reaction was stopped by washing the wells and spots were counted under a stereomicroscope.

### P. berghei challenge

Sporozoites of *P*. *berghei* (ANKA strain clone 1) were obtained from laboratory reared female *Anopheles stephensi* mosquitoes maintained at 18 °C for 20-25 days after feeding on infected mice. Salivary glands from the mosquitoes were collected by dissection and placed in a tissue homogeniser with RPMI 1640 (Sigma) to release the sporozoites, which were then counted using a haemocytometer. Mice were challenged by injection of 2000 sporozoites into the tail vein. Infection was determined by the presence of ring forms in Giemsa stained blood smears taken 7 and 9 days post challenge. If blood-stage parasitaemia was observed at two time points the mice were sacrificed. Surviving animals were observed for at least a further three weeks for the development of malaria symptoms.

### RESULTS

### Immunogenicity of single adenovirus immunisations using different routes of administration

Initially the effect of the route of administration on the ability of Ad-PbCS to induce pb9-specific IFN-γ secreting T cells was tested.

Rodrigues et a2. (1997) J. Immunol. 158(3): 1268-74 had found high levels of malaria-specific CD8+ T cells were induced after i.m. and s.c. immunisation, but not i.v., i.p. or i.n. The inventors did not test i.v. or i.p. as these are not suitable routes for a prophylactic vaccine to be used in humans, but included i.d. and "gene paint" groups - simple administration of recombinant adenovirus onto the skin (known as gene painting), previously shown to be able to induce an immune response against the antigen expressed by the virus [Tang et al., (1997) Nature 388 (6644): 729-30].

Groups of mice received a single immunisation of 10⁷ pfu Ad-PbCS and splenocytes were tested for peptide-specific IFN-γ secreting T cells after 14 days.

The numbers of peptide-specific IFN-γ secreting T cells (Figure 1) detected after i.m. or i.d. immunisation were somewhat higher than those detected after a single immunisation with i.m. DNA, and slightly lower than those detected after i.m. MVA [Gilbert et al., (1999) Biol. Chem. 380(3): 299-303]. Intranasal or s.c. immunisation produced very low numbers of peptide-specific IFN-γ secreting T cells, and none could be' detected in the "gene-paint" group. Intradermal immunisation was used for all subsequent experiments.

### Immunogenicity of different prime/boost immunisations

Figure 2 shows the numbers of peptide-specific IFN-γ secreting T cells detected in the spleens of immunised mice receiving a priming immunisation on day 0 and a boosting immunisation on day 14.

Using the same vaccine to prime and boost resulted in an increase in specific CD8+ T cells, but a far greater increase was seen after heterologous boosting. DNA does not boost an existing response. However the combination of adenovirus priming and MVA boosting resulted in extremely high numbers of peptide specific CD8+ T cells. In addition to being able to prime a response that could be boosted to such high levels, Ad-PbCS was able to boost a response that had been primed by DNA or MVA.

### Immunogenicity of triple combination immunisations

Heterologous priming and boosting is clearly much more effective than using the same vaccine repeatedly. Three different vaccines were employed sequentially. DNA vaccines do not boost, so two combinations were possible, and employed: DNA/Ad/MVA and DNA/MVA/Ad. Groups of mice were immunised at 10 day intervals and splenocytes were tested 10 days after the final immunisation.

As in the previous experiment high numbers of peptide-specific IFN-γ secreting T cells were detected after DNA/MVA, Ad/MVA, MVA/Ad and DNA/Ad immunisations (Figure 3). However these numbers were not increased to the same extent (three to tenfold) when a third heterologous boosting immunisation was given.

### Protection against infectious challenge

Several of the prime/boost combinations employing adenovirus as either a priming or boosting agent had resulted in high numbers of peptide-specific IFN-γ secreting T cells that would be expected to protect mice against challenge with *P*. *berghei* sporozoites. Therefore groups of eight to eleven mice were immunised with a number of different heterologous prime/boost combinations and challenged with 2000 infectious *P*. *berghei* sporozoites two weeks after the boosting immunisation. The results are shown in Table 1.

Intradermal administration of adenovirus followed by i.d. MVA completely protected the immunised mice. In earlier *P. berghei* challenge experiments it was found that i.m. DNA followed by i.d. MVA gave a high level of protection, but that complete protection was only achieved when the MVA was administered intravenously [Schneider et al., (1998) Nat. Med. 4(4): 397-402]. However using adenovirus priming and MVA boosting, both vaccines could be given intradermally.with no loss of protection. MVA priming and adenovirus boosting also resulted in a high level of protection, whereas two subsequent adenovirus immunisations did not. Adenovirus also boosted responses primed by DNA or Ty-VLPs resulting in levels of protection comparable to that obtained by DNA priming and i.d. MVA boosting.

**TABLE 1**

| | | | | |
|---|---|---|---|---|
| Protection of mice immunised with different heterologous prime/boost combinations. DNA was given intramuscularly (50 µg dose). MVA (10⁶ ffu dose) adenovirus (10⁷ pfu dose) and Ty VLPs (100 µg dose) were administered intradermally unless otherwise stated. The priming immunisation was given on day 0, the boosting immunisation on day 14, and the challenge carried out on day 28. | | | | |

| Prime | Boost | No. infected | No. Challenged | % Protection. |
|---|---|---|---|---|
| DNA | MVA | 5 | 10 | 50 |
| Ad | MVA | 0 | 10 | 100 |
| MVA | Ad | 2 | 10 | 80 |
| Ad | Ad | 7 | 8 | 13 |
| DNA | Ad | 5 | 11 | 55 |
| MVA | MVA | 5 | 8 | 38 |
| Ty (i.v.) | Ad | 7 | 10 | 30 |
| Ty (i.v.) | MVA (i.v.) | 1 | 11 | 91 |
| Ty | Ad | 4 | 10 | 60 |
| naive | | 8 | 10 | 20 |

Additional statements of the invention are as follows:
1. A method of boosting a CD8+ T cell immune response to an antigen in an individual, the method including provision in the individual of a replication-deficient adenoviral vector including nucleic acid encoding the antigen or a CD8+ T cell epitope of said antigen operably linked to regulatory sequences for production of said antigen or epitope in the individual by expression from the nucleic acid, whereby a CD8+ T cell immune response to the antigen previously primed in the individual is boosted.
2. A method of inducing a CD8+ T cell immune response to an antigen in an individual, the method comprising administering to the individual a priming composition comprising the antigen or a CD8+ T cell epitope of said antigen or nucleic acid encoding said antigen or epitope and then administering a boosting composition which comprises a replication-deficient adenoviral vector including nucleic acid encoding said antigen or epitope operably linked to regulatory sequences for production of said antigen or epitope in the individual by expression from the nucleic acid.
3. A method according to statement 2, wherein the priming composition comprises DNA encoding said antigen or epitope.
4. A method according to statement 2, wherein the priming composition comprises recombinant Ty-VLP.
5. A method according to statement 2, wherein the priming composition comprises Modified Virus Ankara (MVA).
6. A method according to any one of statements 1 to 5, further comprising administration of another, different boosting composition comprising said antigen or epitope.
7. A method according to any one of statements 1 to 6, wherein the boosting composition is administered intradermally.
8. A method according to any one of statements 1 to 7 wherein the boosting composition is administered intramuscularly.

## Claims

1. Use of a replication-deficient adenoviral vector encoding an antigen or a CD8+ T cell epitope of said antigen, in the manufacture of a medicament for treating an individual in which a CD8+ T cell immune response to the antigen is of therapeutic or prophylactic benefit, wherein the medicament is for administration to such an individual to boost in the individual a CD8+ T cell immune response to the antigen following prior administration of a priming composition comprising said antigen or epitope or nucleic acid encoding said antigen or epitope.

2. Use according to claim 1 wherein the priming composition comprises DNA encoding said antigen or epitope.

3. Use according to claim 1 wherein the priming composition comprises recombinant Ty-VLP.

4. Use according to claim 1 wherein the priming composition comprises Modified Virus Ankara (MVA).

5. Use according to any one of claims 1 to 4 wherein the medicament is a boosting composition for administration prior to administration of another, different boosting composition comprising said antigen or epitope.

6. Use according to any one of claims 1 to 4 wherein the medicament is a boosting composition for administration following administration of another, different boosting comprising said antigen or epitope.

7. Use according to any one of claims 1 to 6 wherein the medicament is for intradermal administration.

8. Use according to any one of claims 1 to 6 wherein the medicament is for intramuscular administration.
